# EUROPEAN PATENT APPLICATION

(11) **EP 4 382 090 A1**
(43) Date of publication of application: **12.06.2024**
(21) Application number: 22212127.9
(22) Date of filing: 08.12.2022
(51) Int. Cl.: A61K 8/68, A61K 8/99, A61Q 19/00, A61K 31/164, A61K 31/23, A61K 35/742, A61P 17/00, A61P 17/06, A61P 17/08, A61P 17/10

(54) **COSMETICAL AND PHARMACEUTICAL COMPOSITIONS CONTAINING BACILLUS STRAINS OR FERMENTATION BROTHS THEREOF**

(71) Applicant: Evonik Operations GmbH, 45128 Essen (DE)
(72) Inventor: VOSS, Michaela, 58456 Witten (DE); HÜSER, Andrea, 33615 Bielefeld (DE); SCHILD, Jennifer, 42653 Solingen (DE); PELZER, Stefan, 33335 Gütersloh (DE); HAKKAART, Xavier, 33615 Bielefeld (DE); MACZKIEWITZ, Ursula, 45219 Essen (DE); MAUS, Lisa, 47228 Duisburg (DE); DIETL, Claudia, 45359 Essen (DE)
(74) Representative: Evonik Patent Association

(57) **Abstract**

The present invention relates to cosmetical and pharmaceutical compositions containing Bacillus strains or fermentation broths thereof and to methods of producing and using such strains.

## Description

The present invention relates to cosmetical and pharmaceutical compositions containing Bacillus strains or fermentation broths thereof and to methods of producing and using such compositions.

Many Bacillus strains are known to be suitable as probiotically active food and feed ingredients, wherein their useability as food and feed ingredients is primarily due to specific enzymatic and growth-inhibiting activities like growth-inhibiting effects against typical human and animal pathogens of the human or animal gut like Clostridia or *E. coli.*

On the other hand, a few bacteria, in particular Lactobacilli and Bifidobacteria, have been disclosed to be suitable as probiotically active substances in cosmetic compositions. Further, for several Bacillus strains it has been shown that they possess growth-inhibiting activity against pathogenic skin bacteria, which makes them suitable as probiotically active substances for cosmetic applications, as well. Thus, WO 2022/119895 discloses *B. velezensis* strains with growth-inhibiting activity against Staphylococci and against Malassezia, so that they can be used in the treatment of dandruff. Further, CN 115044505A discloses a *B. velezensis* strain which produces lipopeptides with growth-inhibiting activity against Propionibacterium acnes.

Inflammation of the skin ("dermatitis") is caused by noxious bacteria or fungi, e.g., C. acnes or S. aureus, which reside on the skin, but proliferate strongly under specific conditions. Uncontrolled proliferation of such bacteria or fungi can cause "impure skin" or acne vulgaris, so that under such conditions said microorganisms or their uncontrolled proliferation are classified as pathogenic.

The bacterial microbiome, on the other hand, also comprises species which do not cause harm and by virtue of their continuous growth keep the pathogenic bacterial species in check, thereby performing an important protective function. The coagulase-negative staphylococcus *S. epidermidis* is an important representative of such a species. In general, the term "saprophyte" is used to designate such benign, protective bacterial species.

Antibacterial agents which target skin bacteria unselectively, such as those normally used to prevent and combat acne in commercial cosmetical products, not only kill pathogenic skin germs but also beneficial skin germs, thus disturbing the biological equilibrium, also called eubiosis, on the skin which can give rise to a variety of undesirable consequences.

There is therefore a need for an agent that selectively fosters, on the applied skin area, the growth and/or the survival of the desired germs of the skin microbiome as opposed to the growth and/or the survivability of the pathogenic microbes present in the skin microbiome. Surprisingly, this task could be solved by the fermentation broths of the invention, in particular by the fermentation broths of Bacillus strain CECT 5940 and *Bacillus subtilis* DSM 32315. Further, it was surprisingly found according to the invention, that a mixture of Bacilli fermentation broths with ceramides effectively enhances the growth-inhibiting activity against pathogenic *C. acnes* bacteria.

In a preferred embodiment of the invention the Bacilli strains, fermentation broths and compositions are able to inhibit the growth of at least one pathogenic skin bacterium, in particular of *C. acnes,* while they do have a much weaker growth-inhibiting effect on beneficial skin bacteria, in particular on the growth of *S. epidermidis.*

In a very preferred embodiment of the invention the Bacilli strains, fermentation broths and compositions are able to inhibit the growth of at least one pathogenic skin bacterium, in particular of *C. acnes,* while they do not have a negative effect or even a positive effect on the growth of beneficial skin bacteria, in particular on the growth of *S. epidermidis,* thus resulting in a very beneficial modulation of the skin microbiome.

A first subject of the present invention is therefore a cosmetical composition containing at least one Bacillus strain or a fermentation broth thereof and at least one ceramide, wherein the composition is preferably a topical skin care or a dermatological composition.

A further subject of the present invention is therefore also a pharmaceutical composition for topical or dermatological applications, in particular for treating or preventing acnes vulgaris, dermatitis, Rosaceae or Couperose, containing at least one Bacillus strain or a fermentation broth thereof and at least one ceramide.

A further subject of the present invention is therefore also a cosmetical composition containing the *Bacillus velezensis* strain CECT 5940 (which is also known as *B. amyloliquefaciens* CECT 5940 strain) or a mutant thereof or a fermentation broth of such strains, wherein the mutant has a sequence identity of at least 99 %, preferably at least 99.5 %, more preferably at least 99.9 % with respect to the genomic DNA of the strain CECT 5940, wherein the composition is preferably a topical skin care or a dermatological composition and wherein the composition preferably comprises at least one ceramide. The strain *B. velezensis*/*B. amyloliquefaciens* CECT 5940 was deposited at the Spanish Type Culture Collection, Parc Cientific Universitat de Valencia, C/ Catedrático Agustin Escardino, 9, 46980 Paterna (Valencia), Spain.

A further subject of the present invention is therefore also a cosmetical composition containing the *B. subtilis* strain DSM 32315 or a mutant thereof or a fermentation broth of such strains, wherein the mutant has a sequence identity of at least 99 %, preferably at least 99.5 %, more preferably at least 99.9 % with respect to the genomic DNA of the strain DSM 32315, wherein the composition is preferably a topical skin care or a dermatological composition and wherein the composition preferably comprises at least one ceramide. The strain *B. subtilis* DSM 32315 was deposited in accordance with the regulations of the Budapest Treaty at the DSMZ-German Collection of Microorganisms and Cell Cultures GmbH, Inhoffenstraße 7B, 38124 Braunschweig, Germany.

A further subject of the present invention is therefore also a pharmaceutical composition for topical or dermatological applications, in particular for treating or preventing acnes vulgaris, dermatitis, Rosaceae or Couperose, containing the *B. velezensis*/*B. amyloliquefaciens* strain CECT 5940 or a mutant thereof or a fermentation broth of such strains, wherein the mutant has a sequence identity of at least 99 %, preferably at least 99.5 %, more preferably at least 99.9 % with respect to the genomic DNA of the strain CECT 5940, wherein the composition preferably comprises at least one ceramide.

A further subject of the present invention is therefore also a pharmaceutical composition for topical or dermatological applications, in particular for treating or preventing acnes vulgaris, dermatitis, Rosaceae or Couperose, containing the *B. subtilis* strain DSM 32315 or a mutant thereof or a fermentation broth of such strains, wherein the mutant has a sequence identity of at least 99 %, preferably at least 99.5 %, more preferably at least 99.9 % with respect to the genomic DNA of the strain DSM 32315, wherein the composition preferably comprises at least one ceramide.

A further subject of the present application is therefore also the use of at least one Bacillus strain, fermentation broth or composition according to the invention for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin.

A further subject of the present application is therefore also the use of the *Bacillus velezensis*/*B. amyloliquefaciens* strain CECT 5940 for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or of increasing the barrier function of skin.

A further subject of the present application is therefore also the use of the *Bacillus subtilis* strain DSM 32315 for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin.

A further subject of the present application is therefore also the combined use of at least one Bacillus strain or of a fermentation broth thereof and of at least one ceramide for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin.

A further subject of the present application is therefore also the combined use of a Bacillus strain or of a fermentation broth thereof and of at least one ceramide for preparing a topical or pharmaceutical composition for treating the human skin, in particular for preparing a topical or pharmaceutical composition for treating or preventing diseases associated with pathogenic C. acnes and/or for treating or preventing acne vulgaris, dermatitis, Rosaceae or Couperose.

A further subject of the present application is therefore also the non-therapeutical use of a Bacillus strain or of a fermentation broth thereof or of a composition according to the invention for treating the skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin.

A further subject of the present invention is therefore also a non-therapeutical method for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin, wherein a Bacillus strain or a fermentation broth thereof or a cosmetical composition according to the invention is applied to human skin.

A further subject of the present invention is therefore also a non-therapeutical method for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin, wherein the Bacillus strain CECT 5940 or a mutant thereof or a fermentation broth of such strains or a cosmetical composition containing such a strain or fermentation broth is applied to human skin, wherein the mutant has a sequence identity of at least 99 %, preferably at least 99.5 %, more preferably at least 99.9 % with respect to the genomic DNA of the strain CECT 5940.

A further subject of the present invention is therefore also a non-therapeutical method for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin, wherein the Bacillus strain DSM 32315 or a mutant thereof or a fermentation broth of such strains or a cosmetical composition containing such a strain or fermentation broth is applied to human skin, wherein the mutant has a sequence identity of at least 99 %, preferably at least 99.5 %, more preferably at least 99.9 % with respect to the genomic DNA of the strain DSM 32315.

A further subject of the present invention is therefore also a therapeutical method of treating or preventing diseases of the human skin, in particular of treating or preventing diseases associated with pathogenic *C. acnes* and/or of diseases selected from acne vulgaris, dermatitis, Rosaceae or Couperose, wherein a Bacillus strain or a fermentation broth thereof or a pharmaceutical composition according to the invention is applied to human skin.

A further subject of the present invention is therefore also a Bacillus strain or a fermentation broth thereof or of a composition according to the invention for use in a method of treating or preventing a disease of the human skin, in particular of treating or preventing a disease associated with pathogenic *C. acnes* and/or of treating or preventing acne vulgaris, dermatitis, Rosaceae or Couperose.

A further subject of the present invention is therefore also a method of preparing a cosmetical or pharmaceutical composition according to the invention, comprising the following steps:
a) Providing a fermentation broth of Bacillus cells with growth-inhibiting activity against skin pathogens, in particular *C. acnes,* wherein the Bacillus cells preferably have no growth-inhibiting activity against *S. epidermidis,*
b) Killing all or the major part of the Bacillus cells, preferably by heat treatment and/or pH shift,
c) Mixing the fermentation broth thus obtained with substances suitable for cosmetical skin applications.

The composition according to the invention is preferably a topical skin care composition.

According to the invention the term "skin" refers preferably to the skin itself, particularly to the human skin, but may also refer to the mucosa and skin adnexa, in so far that they include living cells, particularly hair follicles, hair roots, hair bulbs, the ventral epithelial layer of the nail bed (lectulus) as well as sebaceous glands and perspiratory glands.

The Bacillus strain is according to the invention preferably selected from the species *B. subtilis, B. amyloliquefaciens, B. velezensis, B. licheniformis, B. paralicheniformis* and *B. pumilus,* more preferably from the species *Bacillus subtilis, Bacillus velezensis* or *Bacillus amyloliquefaciens,* above all it is of the species *Bacillus subtilis* or *Bacillus velezensis,* particularly preferred of the species *B. velezensis.*

The term "fermentation broth" according to the invention relates to the product of a cultivation of Bacilli in a suitable fermentation medium. Methods for producing such fermentation broths are well known to those skilled in the art. The fermentation broths of the present invention can for example be obtained by culturing the strains by using the media, conditions and methods as described in US 6,060,051, EP 0287699, US 2014/0010792 or in the FAO Report 179 (2016): "Probiotics in Animal Nutrition". Conventional large-scale microbial culture processes include submerged fermentation, solid state fermentation, or liquid surface culture. Typically, fermentation is carried out, until a certain cell density is reached like an optical density (OD 600) of about 1 to 5, more preferably 3 to 5, above all 4 to 5. The fermentation broths such obtained can be used for the purposes of the invention directly. Alternatively, the fermentation broths thus obtained can also be treated in different ways, e.g., by concentrating or drying of the fermentation broths and/or by inactivating or removing the bacterial cells completely or in part and/or by lysing the bacterial cells or combinations of such methods.

Inactivation of the cells can be carried out e.g. by heat treatment and/or by adjusting an acid or an alkaline pH. Heat inactivation of the cells is preferably carried out by increasing the temperature to between 60°C and 80°C and incubation for at least 30 minutes. pH inactivation at acid pH is preferably carried out by lowering the pH to at least 3 by addition of an acid like 5M H₂SO₄ and incubation for at least 30 minutes, preferably at least one hour. pH inactivation at alkaline pH is preferably carried out by increasing the pH to at least 10 and incubation for at least 30 minutes, preferably at least one hour. Alternatively, inactivation of the cells may also be carried out by applying gamma- or UV-irradiation. In a preferred embodiment of the invention a fermentation broth is used, in which at least 90 %, more preferably at least 95 or 99 %, in particular all bacterial cells are inactivated. Correspondingly, in a preferred embodiment of the invention a fermentation broth is used which contains no viable cells. Such preparations are also denoted as "paraprobiotics". The fermentation broths of the invention, from which the cells have been removed, i.e. the cell-free supernatant and metabolites containing fractions of the fermentation broth, are also denoted as "postbiotics", "metabiotics" or "biogenics".

According to the invention, it is further preferably prevented that the bacterial cells of the fermentation broth transform into spores, preferably by interrupting the cultivation process in time. Thus, in a preferred embodiment of the invention, less than 10 %, more preferably less than 5 %, of the cells in the fermentation broth are present as spores, wherein due to the heat and/or pH inactivation the remaining spores are preferably non-viable. Very preferably the fermentation broth does not contain any spores, at all.

Separation of the microorganisms from the fermentation broth can be carried out for example by centrifugation, flotation, filtration, particularly ultrafiltration or microfiltration and/or decanting.

Drying of the fermentation broth can be carried out for example by freeze-drying, spray drying, vacuum drying, tray drying, drum drying, fluidized bed drying or spray granulation of the fermentation broth.

Lysis of the cells may be carried out mechanically, chemically and/or enzymatically, in particular by osmotic shock, heat shock, sonication, homogenization or shearing by e.g. applying a French press.

Cell containing fermentation broths of the invention preferably contain cells in an amount of 1×10³ to 1×10¹¹ CFU, more preferably in an amount of 1×10⁵ to 1×10¹⁰ CFU, above all in an amount of 1×10⁷ to 1×10⁹ CFU. As in a preferred embodiment of the invention the fermentation broths due to inactivation of the cells do not contain viable cells or only a very small amount, the term CFU (colony forming units) relate to the fermentation broth before inactivation of the cells.

Preferably according to the invention always an effective amount of the strains, fermentation broths and compositions of the invention is used in the embodiments of the invention. The term "effective amount" refers to an amount which effects at least one beneficial effect to the human skin, in particular with respect to the features as already mentioned before, in comparison to a human skin that has not been exposed to the strains, fermentation broths and compositions of the invention, but may have been exposed to the same treatment and/or to the same cosmetical composition depleted by such strains or fermentation broths.

The cosmetic or pharmaceutical composition according to the invention can be in any form suitable for application, e.g., a soap, a lotion, a serum, a spray, a jelly, a cream, a gel, a paste, a pomade, a balm, an ointment, a foam, a mousse, an emulsion, a stick, a patch, a powder, a cleaning fluid or cleaning milk, a deodorant, an anti-perspirant, a salve, a hair conditioner or a shampoo. The composition can also be applied in a mask or in a band-aid, particularly in a gel reservoir mask or band-aid or matrix mask or band-aid.

The compositions of the invention preferably have a pH in the range of between 3 to 8, in particular in the range of 4.0 to 8.0, more preferably in the range of 4.5 to 7.4, particularly preferably in the range of 5.0 to 7.2. The pH of the composition is preferably determined at 22°C after stirring for five minutes using a pH electrode calibrated in accordance with ISO 4319 (1977).

The application area can be the skin of any part of the body, particularly the facial skin, the scalp, the skin on hands and feet, the skin under the arms, as well as the mucous membrane, wherein the facial skin is particularly preferred.

The compositions of the invention preferably comprise at least one ceramide. "Ceramide" according to the invention refers to acylated sphingoid bases, wherein the sphingoid bases are preferably selected from sphingosine, sphinganine, 6-hydroxysphingosine and phytosphingosine.

The ceramide is according to the invention preferably selected from ceramide NP, ceramide AP, ceramide EOP, ceramide NDS, ceramide ADS, ceramide EODS, ceramide NS, ceramide AS, ceramide EOS, ceramide NH, ceramide AH, ceramide EOH and mixtures thereof, more preferably from ceramide NP, ceramide AP, ceramide NS, ceramide EOP, ceramide EOS and mixtures thereof, above all from ceramide NP, ceramide AP and mixtures thereof. The ceramide nomenclature is commonly used and is described in more detail in Farwick et al. Developments in Ceramide Identification, Synthesis, Function and Nomenclature, Cosmet Toil 2009; 124: 63-72.

The composition according to the invention is preferably an emulsion, in particular a W/O or an O/W emulsion. This means that the composition contains preferably at least one surface-active substance as emulsifier or as dispersion agent. Emulsifiers act at the interphase to produce water or oil-stable adsorption layers that protect the dispersed droplets against coalescence and thereby stabilize the emulsion. Thus, emulsifiers, like surfactants, are composed of hydrophobic and hydrophilic molecular moieties. Hydrophilic emulsifiers preferably form O/W emulsions and hydrophobic emulsifiers preferably form W/O emulsions. An emulsion is understood to mean a dispersion of a liquid in the form of droplets in another liquid using an energy input to afford interphases stabilized with surfactants. The choice of this emulsifying surfactant or emulsifier depends on the materials being dispersed and the respective external phase as well as the fineness of the emulsion.

The composition according to the invention preferably comprises non-ionic emulsifiers, more preferably a polyglycerol carboxylic acid ester or mixtures of such compounds. The polyglycerol group of the polyglycerol carboxylic ester has preferably a degree of polymerization of 2.0 to 25, more preferably of 2.5 to 20, in particular of 3.0 to 15, and the carboxylic acid group is preferably a fatty acid, in particular having 12 to 26, preferably 14 to 24, more preferably 16 to 22 carbon atoms. The compositions of the invention preferably comprise at least two different polyglycerol carboxylic esters, wherein the at least two different compounds preferably vary with respect to the degree of polymerization as well as with respect to the carboxylic acid group.

Owing to its polymeric property, the polyglycerol is a statistical mixture of various compounds. Polyglycerol may have ether bonds formed between two primary, one primary and one secondary or else two secondary positions of the glycerol monomers. For this reason, the polyglycerol base skeleton does not usually consist exclusively of linearly linked glycerol units, but may also comprise branches and rings. Further, besides polyglycerol also monomeric glycerol may be present in the polyglycerol carboxylic acid esters to a certain extent. This has to be taken into account for the purposes of calculating amounts, masses and numerical values of the polyglycerol carboxylic acid esters. The values insofar do always relate to average numbers. For details see, for example, *"*Original synthesis of linear, branched and cyclic oligoglycerol standards", Cassel et al., J. Org. Chem. 2001, 875-896. For further preferred characteristics of the polyglycerol carboxylic acid esters as used according to the invention see also WO 2022/073909.

The emulsifiers usable according to the invention may also be selected from addition products of 4 to 30 moles ethylene oxide and/or 0 to 5 moles propylene oxide on linear C8-C22-fatty alcohols, on C12-C22-fatty acids or on C8-C15-alkylphenols; C12-C22 fatty acid mono and diesters of addition products of 1 to 30 moles ethylene oxide on C3-C6 polyols, preferably on glycerin; linear and branched C8-C30 fatty acids and their Na, K, ammonium, Ca, Mg and Zn salts; fatty acid esters of sugars and sugar alcohols such as sorbitol; ethylene oxide and polyglycerin addition products on methylglucoside fatty acid esters, fatty acid alkanolamides or fatty acid glucamides; polyglycerin and polyglycerin derivatives like polyglyceryl-2-dipolyhydroxy stearate or polyglyceryl-3-diisostearate; C8-C22 alkyl mono- and oligoglycosides and their ethoxylated analogs, wherein the degree of oligomerization is preferably 1.1 to 5, in particular 1.2 to 2.0, and glucose is preferred as the sugar component; mixtures of alkyl(oligo) glucosides and fatty alcohols; addition products of 5 to 60 moles ethylene oxide on castor oil or hydrogenated castor oil; partial esters of polyols containing 3-6 carbon atoms with C8-C22 fatty acids; phospholipids like lecithins or phosphatidyl cholines (PC) which are in particular obtainable from egg yolk or plant seeds (e.g. soya beans); and sterols (sterine), wherein "sterols" are understood to mean a group of steroids, which carry a hydroxyl group on carbon atom 3 of the steroid skeleton and are isolated either from animal tissue (zoosterols), vegetal fats (phytosterols) or fungi like yeasts (mycosterols). Examples of zoosterols are cholesterol and lanosterol. Examples of suitable phytosterols are beta-sitosterol, stigmasterol, campesterol and ergosterol.

In a particular embodiment of the invention, at least one non-ionic emulsifier with a HLB value of 8 or below is comprised (according to the definition of HLB value shown in the Römpp-Lexikon Chemie (Eds.: J. Falbe, M. Regitz), 10th edition, Georg Thieme Verlag Stuttgart, New York, (1997), page 1764). Exemplary suitable emulsifiers of this type are compounds of the general formula R<1> -O-R<2> , in which R<1> is a primary linear alkyl, alkenyl or acyl group having 20 to 30 carbon atoms and R<2> is hydrogen, a group of formula -(CnH2nO)x-H with x=1 or 2 and n=2 to 4 or a polyhydroxyalkyl group with 4 to 6 carbon atoms and 2 to 5 hydroxy groups. A particularly preferred emulsifier of formula R<1> -O-R<2> is a behenyl or erucyl derivative, in which R<1> represents a primary linear alkyl, alkenyl or acyl group having 22 carbon atoms.

Further preferred suitable emulsifiers with a HLB value of 8 and below are the addition products of 1 or 2 moles ethylene oxide or propylene oxide on behenyl alcohol, erucyl alcohol, arachidyl alcohol or also on behenic acid or erucic acid. Monoesters of C16-C30 fatty acids with polyols such as e.g. pentaerythreitol, trimethylol propane, diglycerin, sorbitol, glucose or methylglucose are also suitable and preferred. Examples of such products are e.g. sorbitol monobehenate or pentaerythreitol monoerucate.

In another particular embodiment of the invention, at least one ionic emulsifier, selected from anionic, zwitterionic, ampholytic and cationic emulsifiers, is comprised instead or in addition to the non-ionic emulsifiers as mentioned before. Preferred anionic emulsifiers are alkyl sulfates, alkyl polyglycol ether sulfates and ether carboxylic acids with 10 to 18 C atoms in the alkyl group and up to 12 glycol ether groups in the molecule sulfosuccinic acid mono and dialkyl esters with 8 to 18 C atoms in the alkyl group and sulfosuccinic acid mono-alkylpolyoxyethyl esters with 8 to 18 C atoms in the alkyl group and 1 to 6 oxyethylene groups, monoglyceride sulfates, alkyl and alkenyl ether phosphates as well as condensates of protein and fatty acids. Zwitterionic emulsifiers carry at least a quaternary ammonium group and at least one -COO<-> - or SO3<-> group in the molecule. Particularly suitable zwitterionic emulsifiers are the so-called betaines such as N-alkyl-N,N-dimethylammonium glycinates, N-acyl-aminopropyl-N,N-dimethylammonium glycinates and 2-alkyl-3-carboxymethyl-3-hydroxyethyl-imidazolines, each having 8 to 18 carbon atoms in the alkyl or acyl group, as well as cocoacylaminoethylhydroxyethylcarboxymethyl glycinate.

Ampholytic emulsifiers comprise, apart from a C8-C24 alkyl or acyl group, at least one free amino group and at least one COOH or SO3H group in the molecule, and are able to form internal salts. Examples of suitable ampholytic emulsifiers are N-alkylglycines, N-alkylaminopropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids each with about 8 to 24 carbon atoms in the alkyl group.

The compositions according to the invention preferably comprise emulsifiers, in particular non-ionic emulsifiers, above all polyglycerol carboxylic acids, in quantities of 0.1 to 25 wt. %, more preferably 0.5 to 15 wt. %, above all in quantities of 2 to 8 wt. % or 4 to 8 wt.-%, based on the total composition.

In a very preferred embodiment of the invention, the composition according to the invention is a lamellar composition, wherein this lamellar composition forms the basis for the preparation of skinfriendly cosmetical compositions, wherein the lamellar composition is used for the preparation of cosmetical compositions preferably in an amount of from 1 to 5 wt.-%, corresponding to a dilution factor of 1 : 20 to 1 : 100. The cosmetical compositions thus obtained are also a very preferred embodiment of the invention.

The lamellar composition preferably contains at least one fermentation broth, at least one ceramide and at least one polyglycerol carboxylic acids as mentioned before, wherein the fermentation broth is preferably contained in an amount of from 2 to 30 wt.-%, more preferably in an amount of 5 to 25 wt.-%, in particular in an amount of 8 to 20 wt.-%, ceramides are preferably contained in an amount of 0.1 to 5 wt.-%, more preferably in an amount of 0.2 to 4 wt.-%, in particular in an amount of 0.5 to 2.5 wt.-%, and polyglycerol carboxylic acids are preferably contained in a total amount of 1 to 12 wt.-%, more preferably in an amount of 4 to 8 wt.-%. In the cosmetical compositions as obtained by using such lamellar compositions for preparing the cosmetical compositions, the mentioned compounds are accordingly preferably present in an amount 20 to 100 times lower.

Instead of or in addition to the ceramides, the compositions of the invention may also comprise at least one sphingoid base, wherein the sphingoid base is preferably selected from sphingosine, sphinganine, 6-hydroxysphingosine, N-acetylsphingosine and phytosphingosine, with phytosphingosine being preferred. In a preferred embodiment of the invention a combination of ceramides and phytosphingosines is used in the compositions.

In a further preferred embodiment of the invention, the compositions comprise at least one phospholipid. Phospholipids which are preferably used according to the invention are phosphatidyl cholines (PC), also known as lecithins, phosphatidylethanolamines (PE), also known as cephalins, phosphatidic acid (PA), also known as phosphatidates, phosphatidylserines (PS), phosphoinositides like phosphatidylinositol (PI), phosphatidylinositol phosphate (PIP), phosphatidylinositol bisphosphate (PIP2), phosphatidylinositol trisphosphate (PIP3), phosphosphingolipids like ceramide phosphorylcholines (SPH), ceramide phosphorylethanoloamines (Cer-PE) and ceramide phosphoryllipids. Phospholipids are obtainable from biological sources like plants and animals, in particular from egg yolk, bovine milk, fish eggs or plant seeds (e.g. soya beans, rapeseed or sunflower seeds).

"Sterols" are understood to mean a group of steroids, which carry a hydroxyl group on carbon atom 3 of the steroid skeleton and are isolated either from animal tissue (zoosterols), vegetal fats (phytosterols) or fungi like yeasts (mycosterols). Examples of zoosterols are cholesterol and lanosterol. Examples of suitable phytosterols are beta-sitosterol, stigmasterol, campesterol and ergosterol. Preferred sterols comprised in the composition according to the invention are selected from the group consisting of cholesterol, 7-dehydrocholesterol, potassium cholesterol sulfate, cholesteryl succinate, 25-hydroxy-7-dehydrocholesterol, ergosterol, fucosterol, hopanoids, hydroxysteroid, phytosterols like for example campesterol, sitosterol, stigmasterol, steroids and zoosterols, with cholesterol being especially preferred.

In a further preferred embodiment of the invention, the compositions comprise at least one free fatty acid, wherein the free fatty acid preferably has a chain length of 12 to 40, more preferably 14 to 24, particulary preferably 16 to 22 carbon atoms and wherein the free fatty acid is preferably a saturated fatty acid.

A particularly preferred embodiment of the invention is a composition containing
a) a Bacillus fermentation broth in an amount of 2 to 30 wt.-%, preferably in an amount of 5 to 25 wt.-%, in particular in an amount of 8 to 20 wt.-%, wherein the fermentation broth preferably does not contain viable cells;
b) ceramides in an amount of 0.1 to 5 wt.-%, more preferably in an amount of 0.2 to 4 wt.-%, in particular in an amount of 0.5 to 2.5 wt.-%;
c) polyglycerol carboxylic acids in a total amount of 1 to 12 wt.-%, more preferably in an amount of 4 to 8 wt.-%;
d) preferably at least one phospholipid, more preferably cholesterol or a derivative thereof, in an amount of 0.1 to 3 wt.-%, more preferably in an amount of 0.1 to 2 wt.-%;
e) preferably at least one sphingosine base in an amount of 0.01 to 2 wt.-%, preferably in an amount of 0.02 to 1 wt.-%;
f) preferably at least one free fatty acid in an amount of 0.01 to 3 wt.-%, more preferably in an amount of 0.05 to 2 wt.-%.

A further particularly preferred embodiment of the invention is also a composition containing
a) a fermentation broth of Bacillus strain CECT 5940 or of Bacillus strain DSM 32315 or of a mutant of such strains, preferably in an amount of 2 to 30 wt.-%, more preferably in an amount of 5 to 25 wt.-%, in particular in an amount of 8 to 20 wt.-%, wherein the fermentation broth preferably does not contain viable cells;
b) ceramides in an amount of 0.1 to 5 wt.-%, more preferably in an amount of 0.2 to 4 wt.-%, in particular in an amount of 0.5 to 2.5 wt.-%;
c) preferably polyglycerol carboxylic acids in a total amount of 1 to 12 wt.-%, more preferably in an amount of 4 to 8 wt.-%;
d) preferably at least one phospholipid, more preferably cholesterol or a derivative thereof, in an amount of 0.1 to 3 wt.-%, more preferably in an amount of 0.1 to 2 wt.-%;
e) preferably at least one sphingosine base in an amount of 0.01 to 2 wt.-%, preferably in an amount of 0.02 to 1 wt.-%;
f) preferably at least one free fatty acid in an amount of 0.01 to 3 wt.-%, more preferably in an amount of 0.05 to 2 wt.-%.

A further particularly preferred embodiment of the invention are accordingly also the cosmetical compositions which can be obtained by using such compositions in an amount of 1 to 5 wt.-% and which accordingly contain the compounds as mentioned before preferably in an amount 20 to 100 times lower.

In addition to or instead of the emulsifiers as mentioned above the compositions according to the invention may also comprise foaming, non-ionic, zwitterionic, anionic and/or cationic surfactants as further surface-active ingredients.

Examples of non-ionic surfactants are alkoxylated fatty acid alkyl esters of the formula R<1>CO-(OCH2CHR<2>)xOR<3> in which R<1>CO stands for a linear or branched, saturated and/or unsaturated acyl group with 6 to 22 carbon atoms, R<2> for hydrogen or methyl, R<3> for linear or branched alkyl groups with 1 to 4 carbon atoms and x for numbers from 1 to 20; addition products of ethylene oxide to fatty acid alkanolamides and fatty amines; fatty acid N-alkylglucamides; C8-C22 alkylamine-N-oxides; alkyl polyglycosides corresponding to the general formula RO-(Z)x wherein R stands for a C8-C16 alkyl, Z for sugar and x for the number of sugar units. The alkyl polyglycosides used according to the invention may simply comprise a defined alkyl group R. However normally, these compounds are manufactured from natural fats and oils or mineral oils, in which case the alkyl groups R are present as mixtures corresponding to the starting compounds or to each of the compounds as worked up. Alkyl polyglycosides are particulary preferred, in which R is essentially selected from C8- and C10-alkyl groups, from C12- and C14-alkyl groups, from C8- to C16-alkyl groups or from C12- to C16-alkyl groups.

Any mono or oligosaccharide can be added as the sugar building block Z. Normally, sugars having 5 or 6 carbon atoms as well as the corresponding oligosaccharides are added, for example, glucose, fructose, galactose, arabinose, ribose, xylose, lyxose, allose, altrose, mannose, gulose, idose, talose and sucrose. Preferred sugar building blocks are glucose, fructose, galactose, arabinose and sucrose; glucose is particularly preferred. The inventively usable alkyl polyglycosides comprise an average of 1.1 to 5, preferably 1.1 to 2.0, particularly preferably 1.1 to 1.8 sugar units. The alkoxylated homologs of the cited alkyl polyglycosides can also be used according to the invention. These homologs can comprise on average up to 10 ethylene oxide and/or propylene oxide units per alkyl glycoside unit.

Zwitterionic surfactants are surface-active compounds which carry at least one quaternary ammonium group and at least one -COO<(-)> or SO3<(-)> group in the molecule. Particularly suitable zwitterionic surfactants are the so-called betaines such as the N-alkyl-N,N-dimethylammonium glycinates, for example the cocoalkyldimethylammonium glycinate, N-acylaminopropyl-N,N-dimethylammonium glycinates, for example the cocoacylaminopropyldimethylammonium glycinate, and 2-alkyl-3-carboxymethyl-3-hydroxyethyl imidazolines with 8 to 18 carbon atoms in each of the alkyl or acyl groups, as well as cocoacylaminoethylhydroxyethylcarboxymethyl glycinate. A preferred zwitterionic surfactant is the fatty acid amide derivative, known under the INCI name cocoamidopropyl betaine.

Suitable anionic surfactants according to the invention are all anionic surface-active materials that are suitable for use on the human body. They are characterized by a water solubilizing anionic group, such as e.g. a carboxylate, sulfate, sulfonate or phosphate group and a lipophilic alkyl group containing about 8 to 30 C atoms. In addition, the molecule may contain glycol or polyglycol ether groups, ester, ether and amide groups as well as hydroxyl groups. Exemplary suitable foaming anionic surfactants are, each in the form of the sodium, potassium and ammonium as well as the mono, di and trialkanolammonium salts with 2 to 4 carbon atoms in the alkanol group, acylglutamates of Formula (I), 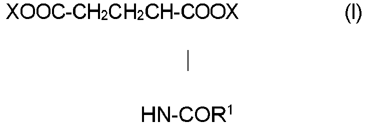
in which R<1>CO stands for a linear or branched acyl group with 6 to 22 carbon atoms and 0, 1, 2 or 3 double bonds and X for hydrogen, an alkali and/or alkaline earth metal, ammonium, alkylammonium, alkanolammonium or glucammonium, for example acylglutamates that derive from fatty acids having 6 to 22, preferably 12 to 18 carbon atoms, such as, for example C12/14- or C12/18-coco fatty acid, lauric acid, myristic acid, palmitic acid and/or stearic acid, particularly sodium N-cocoyl and sodium N-stearoyl-L-glutamate; esters of a hydroxy-substituted di or tricarboxylic acid of the general formula (II), 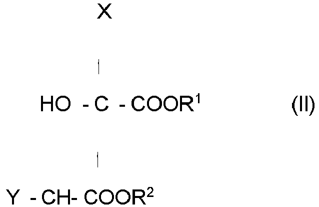
in which X is H or is a -CH2COOR group, Y is H or-OH, with the proviso that if Y is H and if X is CH2COOR, R, R<1> and R<2> , independently of each other, signify a hydrogen atom, an alkali or alkaline earth metal cation, an ammonium group, the cation of an ammonium organic base or a group Z which derives from a polyhydroxylated organic compound selected from the group of etherified (C6-C18)-alkylpolysaccharides having 1 to 6 monomeric saccharide units and/or the etherified aliphatic (C6-C16)-hydroxyalkyl polyols having 2 to 16 hydroxyl groups, with the proviso that at least one of the groups R, R1 and R2 is a group Z; esters of the sulfosuccinic acid of the general formula (III), 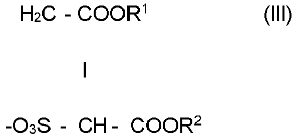
in which R1 and R2, independently of one another signify a hydrogen atom, an alkali or alkaline earth metal cation, an ammonium group, the cation of an ammonium organic base or a group Z which derives from a polyhydroxylated organic compound selected from the group of etherified (C6-C18)-alkylpolysaccharides having 1 to 6 monomeric saccharide units and/or the etherified aliphatic (C6-C16)-hydroxyalkyl polyols having 2 to 16 hydroxyl groups, with the proviso that at least one of the groups R1 or R2 is a group Z;
sulfosuccinic acid mono and dialkyl esters with 8 to 24 carbon atoms in the alkyl group and
sulfosuccinic acid mono-alkylpolyoxyethyl esters with 8 to 24 C atoms in the alkyl group and 1 to 6 ethoxy groups;
esters of tartaric acid and citric acid with alcohols, which represent the addition products of about 2-15 molecules of ethylene oxide and/or propylene oxide on fatty alcohols with 8 to 22 C atoms, linear and branched fatty acids with 8 to 30 C atoms (soaps);
ether carboxylic acids of the formula R-O-(CH2-CH2O)x-CH2-COOH, in which R is a linear alkyl group with 8 to 30 C atoms and x=0 or 1 to 16;
acyl sarcosinates with a linear or branched acyl group having 6 to 22 carbon atoms and 0, 1, 2 or 3 double bonds;
acyl taurates with a linear or branched acyl group having 6 to 22 carbon atoms and 0, 1, 2 or 3 double bonds;
acyl isethionates with a linear or branched acyl group having 6 to 22 carbon atoms and 0, 1, 2 or 3 double bonds;
linear alkane sulfonates with 8 to 24 C atoms;
linear alpha-olefin sulfonates with 8 to 24 C atoms;
alpha-sulfo fatty acid methyl esters of fatty acids with 8 to 30 C atoms;
alkyl sulfates and alkyl polyglycol ether sulfates of the formula R-O(CH2-CH2O)2-SO3X, in which R is a preferably linear alkyl group having 8 to 30 carbon atoms, particularly preferably 8 to 18 carbon atoms, z=0 or 1 to 12, particularly preferably 3, and X is a sodium, potassium, magnesium, zinc, ammonium ion or a monoalkanol-, dialkanol- or trialkanolammonium ion having 2 to 4 carbon atoms in the alkanol groups, wherein a particularly preferred example is zinc cocoyl ether sulfate having an ethoxylation degree of z=3;
mixtures of surface-active hydroxy sulfonates according to DE-A-37 25 030,
sulfated hydroxyalkyl polyethylenes and/or hydroxyalkylene propylene glycol ethers according to DE-A-37 23 354,
sulfonated unsaturated fatty acids with 8 to 24 C atoms and 1 to 6 double bonds according to DE-A-39 26 344,
alkyl- and/or alkenyl ether phosphates of Formula (IV), 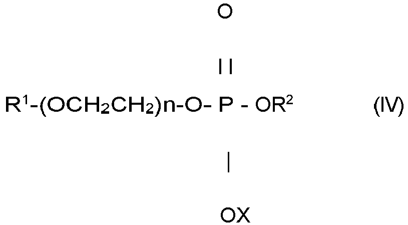
in which R1 preferably stands for an aliphatic hydrocarbon radical with 8 to 30 carbon atoms, R2 stands for hydrogen, a (CH2CH2O)nR1 group or X, n for numbers between 1 and 10 and X for hydrogen, an alkali or alkaline earth metal or NR3, R4, R5, R6, with R3 to R6, independently of each other, standing for a C1, to C4 hydrocarbon group, sulfated fatty acid alkylene glycol esters of Formula R7 CO(AlkO)nSO3M in which R7CO stands for a linear or branched, aliphatic, saturated and/or unsaturated acyl radical with 6 to 22 carbon atoms, Alk for CH2CH2, CHCH3CH2 and/or CH2CHCH3, n for numbers from 0.5 to 5 and M for a cation, like those described in DE-OS 197 36 906.5 and/or monoglyceride sulfates and monoglyceride ether sulfates of Formula (V), 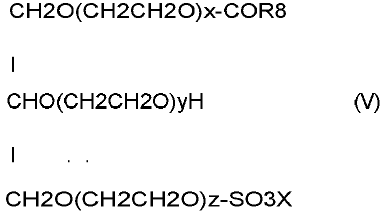
in which R8CO stands for a linear or branched acyl group with 6 to 22 carbon atoms, the sum of x, y and z is 0 or stands for numbers between 1 and 30, preferably 2 to 10, and X stands for an alkali or alkaline earth metal. In the context of the invention, typical examples of suitable monoglyceride (ether) sulfates are the reaction products of lauric acid monoglyceride, cocoa fatty acid monoglyceride, palmitic acid monoglyceride, stearic acid monoglyceride, oleic acid monoglyceride and tallow fatty acid monoglyceride as well as their ethylene oxide adducts with sulfur trioxide or chlorosulfonic acid in the form of their sodium salts. Preferably, monoglyceride sulfates of Formula (VI) are added, in which R8CO stands for a linear acyl group with 8 to 18 carbon atoms.

Instead or in addition to the surfactants as mentioned before, the composition according to the invention may also comprise biosurfactants, wherein according to the invention the term "biosurfactants" is understood to mean all glycolipids produced by fermentation, but also covers glycolipids that are chemically and/or enzymatically modified after fermentation, as long as structurally a glycolipid remains. Raw materials for producing the biosurfactants that can be used are carbohydrates, in particular sugars such as e.g. glucose and/or lipophilic carbon sources such as fats, oils, partial glycerides, fatty acids, fatty alcohols, long-chain saturated or unsaturated hydrocarbons. Where average values are stated hereinbelow, then, unless stated otherwise, these are number- averaged average values. Preferred biosurfactants according to the invention are rhamnolipids, sophorolipids, glucolipids, cellulose lipids, mannosylerythritol lipids and trehalose lipids, in particular rhamnolipids, sophorolipids and glucolipids, most preferably rhamnolipids.

The biosurfactants can be produced as disclosed e.g. in EP 0499434, US 7,985,722, WO 03/006146, JP 60183032, DE 19648439, DE 19600743, JP 01304034, CN 1337439, JP 2006274233, KR 2004033376, JP 2006083238, JP 2006070231, WO 03/002700, FR 2740779, DE 2939519, US 7,556,654, FR 2855752, EP 1445302, JP 2008062179 and JP 2007181789 or the documents cited therein. Suitable biosurfactants can be acquired e.g. from Soliance, France. Preferably, the composition according to the invention contains as biosurfactant at least one compound selected from rhamnolipids, in particular mono-, di- or polyrhamnolipids, glucolipids, in particular mono-, di- or polyglucolipids, and sophorolipids, in particular mono-, di- or polysophorolipids, most preferably rhamnolipids.

In addition, the compositions according to the invention may also comprise at least one protein hydrolyzate or a derivative thereof. According to the invention, the added protein hydrolyzates can be of either vegetal or animal origin. Animal protein hydrolyzates are, for example, elastin, collagen, keratin, silk and milk protein hydrolyzates, which can also be present in the form of their salts. According to the invention, protein hydrolyzates of vegetal origin, e.g. soya, wheat, almond, pea, potatoes and rice protein hydrolyzates, are preferred.

The protein hydrolyzate described above can be substituted with amino acid mixtures, or individual amino acids as well as their physiologically compatible salts. Preferred amino acids for this purpose include for example, glycine, serine, threonine, cysteine, asparagine, glutamine, pyroglutamic acid, alanine, valine, leucine, isoleucine, proline, tryptophan, phenylalanine, methionine, tyrosine, asparaginic acid, glutamic acid, lysine, arginine and histidine as well as the zinc salts and the acid addition salts of these amino acids.

Likewise, it is possible to add derivatives of protein hydrolyzates, e.g. in the form of their fatty acid condensation products.

According to the invention, cationic protein hydrolyzates may also be employed which are obtainable from a variety of biological sources, including without limitation, animals, plants, marine life forms, or from protein hydrolyzates of biotechnological origin. Preferred cationic protein hydrolyzates possess a base protein content of molecular weight of 100 to 25000 daltons, preferably 250 to 5000 daltons. In this context, cationic protein hydrolyzates are understood to include quaternized amino acids and their mixtures. Moreover, the cationic protein hydrolyzates can also be further derivatized. Typical examples of cationic protein hydrolyzates and derivatives thereof are available commercially and include those cited in the "International Cosmetic Ingredient Dictionary and Handbook" (sixteenth edition 2016, The Cosmetic, Toiletry, and Fragrance Association 1101 17th Street, N. W., Suite 300, Washington, D.C. 20036-4702). Examples are cocodimonium hydroxypropyl hydrolyzed collagen, cocodimonium hydroxypropyl hydrolyzed casein, steardimonium hydroxypropyl hydrolyzed collagen, steardimonium hydroxypropyl hydrolyzed hair keratin, lauryldimonium hydroxypropyl hydrolyzed keratin, cocodimonium hydroxypropyl hydrolyzed rice protein, cocodimonium hydroxypropyl hydrolyzed silk, cocodimonium hydroxypropyl hydrolyzed soy protein, cocodimonium hydroxypropyl hydrolyzed wheat protein, cocodimonium hydroxypropyl silk amino acids, hydroxypropyl arginine lauryl/myristyl ether HCI, hydroxypropyltrimonium gelatin. Cationic protein hydrolyzates and derivatives obtained from plants are quite particularly preferred.

The compositions according to the invention comprise the protein hydrolyzates and their derivatives or the amino acids and their derivatives, if present, preferably in quantities of 0.01 to 10 wt. %, based on the total composition. Quantities of 0.1 to 5 wt. %, particularly 0.1 to 3 wt. %, are quite particularly preferred.

In addition, the compositions of the invention may include at least one mono-, oligo- or polysaccharide or derivatives thereof.

Suitable monosaccharides include, without limitation, glucose, fructose, galactose, arabinose, ribose, xylose, lyxose, allose, altrose, mannose, gulose, idose and talose, the desoxysugar fucose and rhamnose as well as amino sugars such as e.g. glucosamine or galactosamine. Glucose, fructose, galactose, arabinose and fucose are preferred.

Suitable oligosaccharides are composed of two to ten monosaccharide units, e.g. saccharose, lactose or trehalose. Saccharose is a particularly preferred oligosaccharide. The use of honey, which comprises mainly glucose and saccharose, is also particularly preferred.

Suitable polysaccharides are composed of more than ten monosaccharide units. Preferred polysaccharides are the starches based on [alpha]-D-glucose units as well as starch degradation products e.g., amylose, amylopectin and dextrin. According to the invention, chemically and/or thermally modified starches, e.g. hydroxypropyl starch phosphate or dihydroxypropyl distarch phosphate. Dextrans as well as their derivatives, e.g. dextran sulfate, are also preferred. Non-ionic cellulose derivatives, such as methyl cellulose, hydroxypropyl cellulose or hydroxyethyl cellulose, as well as cationic cellulose derivatives, Polyquaternium-10 as well as Polyquaternium-24, are similarly preferred. Further preferred examples are polysaccharides from fucose units. Polysaccharides based on amino sugar units, particularly chitins and their deacetylated derivatives, the chitosans, and mucopolysaccharides are particularly preferred. The mucopolysaccharides, preferred according to the invention, include hyaluronic acid and its derivatives, e.g. sodium hyaluronate or dimethylsilanol hyaluronate, as well as chondroitin and its derivatives, e.g. chondroitin sulfate.

In a particular embodiment, the composition according to the invention comprise at least one film forming, emulsion stabilizing, thickening or adhesive polymer, selected from naturally occurring and synthetic polymers which can be cationic, anionic, amphoterically charged or non-ionic. Preferred cationic polymers include polysiloxanes having quaternary groups.

Preferred anionic polymers comprise carboxylate- and/or sulfonate groups and for example acrylic acid, methacrylic acid, crotonic acid, maleic anhydride and 2-acrylamido-2-methylpropane sulfonic acid as monomers. Here, the acidic groups may be fully or partially present as sodium, potassium, ammonium, mono or triethanolammonium salts. Preferred monomers are 2-acrylamido-2-methylpropane sulfonic acid and acrylic acid. Particularly preferred anionic polymers comprise 2-acrylamido-2-methylpropane sulfonic acid as the sole monomer or comonomer, wherein the sulfonic acid group can be totally or partially present as the salt. In this embodiment, it is preferred to use copolymers of at least one anionic monomer and at least one non-ionic monomer. Regarding the anionic monomers, reference is made to the abovementioned substances. Preferred non-ionic monomers are acrylamide, methacrylamide, acrylic acid esters, methacrylic acid esters, vinyl pyrrolidone, vinyl ethers and vinyl esters. Preferred anionic copolymers are acrylic acidacrylamide copolymers and particularly polyacrylamide copolymers with monomers that contain sulfonic acid groups. A particularly preferred anionic copolymer consists of 70 to 55 mole % acrylamide and 30 to 45 mole % 2-acrylamido-2-methylpropane sulfonic acid, wherein the sulfonic acid groups may be fully or partially present as the sodium, potassium, ammonium, mono or triethanolammonium salt. This copolymer can also be crosslinked, wherein the preferred crosslinking agents include polyolefinic unsaturated compounds such as tetraallyloxyethane, allyl sucrose, allyl pentaerythritol, methylene bisacrylamide and sodium acryloyl dimethyl taurate copolymers.

Further preferred anionic homo and copolymers are uncrosslinked and crosslinked polyacrylic acids. Here the preferred crosslinking agents can be allyl ethers of pentaerythritol, of sucrose and of propylene. A particularly preferred anionic copolymer comprises as monomers 80-98% of an unsaturated, optionally substituted C3-6 carboxylic acid or its anhydride as well as 2-20% of an optionally substituted acrylic acid ester of saturated C10-30 carboxylic acids, wherein the copolymer can be crosslinked with the abovementioned crosslinking agents.

Suitable non-ionic polymers include polyvinyl alcohols that can be partially saponified as well as vinyl pyrrolidone/vinyl ester copolymers and polyvinyl pyrrolidones.

The cosmetic compositions according to the invention may also comprise at least one organic or mineral or modified mineral light stabilizer. The light stabilizers are liquid or crystalline substances at room temperature which are able to absorb UV radiation and emit the resulting energy in the form of longer wavelength radiation, for example as heat. Generally, one differentiates between UVA-filters and UVB-filters. Naturally, the UV-A and UV-B filters can also be added as mixtures. According to the invention, it is preferred to add mixtures of filters, if UV filters are used in the compositions.

The compositions of the invention may also comprise further cosmetically active ingredients like glycogen, peptides, amino acids, phenols, polyphenols, vitamins, extracts from plants or algae and cosmetic oils.

Preferred peptides comprised in the composition according to the invention are selected from the group consisting of palmitoyl hexapeptide-12, palmitoyl oligopeptide, palmitoyl pentapeptide-3, palmitoyl tetrapeptide-7, palmitoyl tripeptide-1, collagen, elastin, epidermal growth factor (EGF), epitalon and fibronectin.

Preferred amino acids comprised in the composition according to the invention are selected from the group consisting of proteinogenic amino acids, preferably alanine, valine, isoleucine, leucine, methionine, phenylalanine, tyrosine and tryptophan.

Preferred phenols and polyphenols comprised in the composition according to the invention are selected from the group consisting of resveratrol, quercetin, rutin, ellagic acid, apigenin, phloretin, caftaric acid, caffeic acid phenylethyl ester, pterostilbene, luteolin, fisetin, honokiol, ferulic acid and sinapic acid.

Preferred vitamins comprised in the composition according to the instant invention are selected from the group consisting of coenzyme q10 (ubiquinone), biotin, vitamin A, retin-A, retinoids, retinol, retinaldehyde, retinol palmitate, retinyl ascorbate, retinyl palmitate, retinyl retinoate, vitamin D, cholecalciferol, ergocalciferol, vitamin E, preferably alpha-, beta-, gamma-, and delta-tocopherol, tocopherol, tocopherol acetate, tocopheryl acetate, tocopheryl lineolate, tocopheryl linoleate, tocopheryl linoleate/oleate, tocopheryl nicotinate, tocopheryl succinate, tocotrienols, vitamin K and vitamin C, preferably ascorbyl palmitate.

Preferred extracts from plants or algae comprised in the composition according to the invention are selected from the group consisting of, aloe (especially vera) extract, aesculus hippocastanum extract, calendula officinalis flower extract, centella asiatica extract, bakuchiol, ascophyllum nodosum extract, acemannan, coleus barbatus extract, cranberry seed extract, ginkgo biloba leaf extract, ginseng extract, grapefruit peel extract, green tea extract, hordeum vulgare extract, horse chestnut extract, oryza sativa (rice) extract, pectin, polygonum cuspidatum root extract, pomegranate extract, spirulina extract, squalene, st. john's wort extract, willow bark extract, capsaicin, capsicum extracts, glycyrrhiza glabra extract, madecassoside, rosmarinus officinalis extract, ruscogenine, carotinoids, preferably astaxanthin, β-carotin, canthaxanthin, capsanthin, capsorubin, cryptoxanthin, lutein, luteoxanthin, lycopin and zeaxanthin, chamomilla recutita (matricaria) flower extract, sesamin, pyrus malus fruit extract, baicalein, puerarin, phlorizin, 1,4-dicaffeoylquinic acid, myricetin-3-o-β-rhamnoside, dihydromyricetin, diosmetin, 6-gingerol and mangnolol.

Preferred cosmetic oils comprised in the composition according to the invention are selected from the group consisting of argan oil, almond oil, blackcurrant oil, chia oil, cannabis sativa I. oil, avocado oil (persea gratissima), caprylic/capric triglycerides, cotton seed oil, hemp oil, canola oil, marula oil, peach kernel oil, perilla oil, pomegranate seed oil, pumpkin seed oil, sea buckthorn oil, coconut oil, coconut oil-derived fatty acids, glycine soja oil, helianthus annuus seed oil, jojoba oil, macadamia nut oil, persea gratissima oil, triticum vulgare (wheat) germ oil, grape seed oil, MCT oil, grapefruit oil, ginger oil, butyrospermum parkii (shea) butter, camellia sinensis seed oil, cocoa butter, coconut oil and evening primrose oil.

In a further preferred embodiment of the invention, the composition according to the invention is a liposomal composition, i.e., a composition which contains liposomes. The liposomal composition of the invention preferably contains phospholipids or sterols (sterines) as mentioned before.

In liposomal compositions of the invention, the liposomes have preferably a mean particle size of 15 nm to 800 nm, in particular of 50 nm to 500 nm, more preferably of 60 nm to 350 nm and even more preferably of 80 nm to 240 nm. Photon correlation spectroscopy is preferably employed in order to determine the mean particle size. The measurement is preferably performed using a Zetasizer Nano ZS90, Malvern Instruments Ltd., UK, according to the manufacturer's instruction. The Z-average is the intensity weighted mean hydrodynamic size of the ensemble collection of particles measured by dynamic light scattering (DLS). The Z-average is derived from a Cumulants analysis of the measured correlation curve, wherein a single particle size is assumed and a single exponential fit is applied to the autocorrelation function (see Zetasizer Nano ZS90 User Manual MAN0485-1-1 09 June 2017).

The compositions according to the invention can further comprise at least one additional component selected from the group of emollients, thickeners, viscosity regulators, stabilizers, antioxidants, hydrotropes, solids, fillers, film formers, pearlescence additives, deodorant and antiperspirant active ingredients, insect repellents, self-tanning agents, preservatives, conditioning agents, perfumes, dyes, odour absorbers, superfatting agents, other solvents.

As regards further optional components and the amounts used of these components, reference is made expressly to the relevant handbooks known to those skilled in the art, for example K. Schrader, "Grundlagen und Rezepturen der Kosmetika [Cosmetics - fundamentals and formulations]", 2nd edition, pages 329 to 341, Hüthig Buch Verlag Heidelberg. The amounts of the particular additives are determined by the intended use.

### Working examples

### Example 1: Production of the fermentation broth of B. velezensis/B. amyloliquefaciens CECT 5940 and B. subtilis DSM 32315

Production of the fermentation broth is carried out by inoculating either an LB Kelly fermentation medium with an aliquot of the Bacillus strain CECT 5940 or with an aliquot of the *B. subtilis* strain DSM 32315 and incubating the medium for about 16 hours at 37°C and 200 rpm, until an optical density (OD 600) of 5 was reached. The LB Kelly medium contained 40 g soy peptone, 40 g dextrin 10, 1.8 g KH2PO4, 4.5 g K2HPO4, 0.3 g MgSO4*7H2O and 0.2 ml KellyT trace metal solution per liter, wherein the KellyT trace metal solution contains 25 mg EDTA disodium salt dihydrate, 0.5 g ZnSO4*7H2O, 3.67 g CaCl2*2H2O, 1.25 g MnCl2*4H2O, 0.25 g CoCl2*6H2O, 0.25 g ammonium molybdate, 2.5 g FeSO4*7H2O, and 0.1 g CuSO4*5H2O adjusted to pH 6 with NaOH, 500 ml H2O.

The fermentation broth as obtained contained Bacillus CECT 5940 cells or *B. subtilis* DSM 32315 cells in an amount of about 1×10⁸CFU.

### Example 2: Inactivation of the fermentation broth of Bacillus strain CECT 5940

The fermentation broth as obtained according to example 1 is treated in different ways for inactivating the Bacillus cells as contained in the fermentation broth. Inactivation is carried out either by heat inactivation, pH inactivation or a combination of both. Heat inactivation is carried out by increasing the temperature to either 60°C and incubation for 1 hour or increasing the temperature to 80°C and incubation for 30 min. pH inactivation is carried out by lowering the pH to 3 by addition of 5M H₂SO₄ and incubation for 1 hour. After incubation at increased temperatures and/or lowered pH, 50 ml aliquots of the treated fermentation broth are cooled to a temperature of-20°C for storage.

### Example 3: Testing of the inhibitory activity of the inactivated fermentation broths of Bacillus CECT 5940 and B. subtilis DSM 32315 on C. acnes and on S. epidermidis

The effect of the inactivated fermentation broths of Bacillus strain CECT 5940 and *B. subtilis* DSM 32315 on *Cutibacterium acnes* was measured in a plate-based inhibition assay. *C. acnes* subsp. acnes ATCC 6919 and S. epidermidis ATCC 12228 strains were cultivated anaerobically for 24 h in TSB medium supplemented with 0.2% Tween 80. By measuring the optical density at 600 nm, the C. *acnes* culture was adjusted to an OD of 2.5 and 135µl of this culture was subsequently plated on a TSB plate supplemented with 0.2% Tween 80. Four to five holes with a diameter of 9 mm were stamped into the agar plates and 100 µl of the test substances were put into the holes. After a short time of drying, the plates were incubated for 24 h at 37°C anaerobically. The halo size was determined as the mean value of 2 measurements (vertical and horizontal) minus the diameter of the holes.

**Table 1: Inhibitory activity of inactivated fermentation broths of Bacillus CECT 5940 and B. subtilis DSM 32315 on skin bacteria**

| Skin strain → | C. acnes | S. epidermidis |
|---|---|---|
| *Bacillus* strain ↓ | | |
| Bacillus | 13.8 mm | 0.0 mm |
| CECT 5940 | | |
| B. subtilis | 13.9 mm | 0.0 mm |
| DSM 32315 | | |

As can be seen, the inactivated fermentation broths of Bacillus strain CECT 5940 and *B. subtilis* DSM 32315 selectively inhibit the growth of the pathogenic skin bacterium *C. acnes,* while they do not inhibit the growth of the saprophytic skin bacterium *S. epidermidis.*

### Example 4: Preparation of a composition containing ceramides and the inactivated fermentation broths of Bacillus strain CECT 5940 or B. subtilis DSM 32315

In this example a mixture of the polygyceryl fatty acids Polygyceryl-10 stearate, Polyglycerol-10 palmitate and Polyglyceryl-10 behenate was used emulsifier, as mixtures of different polyglyceryl fatty acids are known to be very beneficial for the skin (see WO 2022/073909). The preparation of the polygyeryl fatty acids was carried out like disclosed in the working examples of WO 2022/073909 (page 9, example 1c).

**Table 2: Composition containing inactivated Bacillus fermentation broths and ceramides**

| Ingredient | Amount [wt.-%] | Phase |
|---|---|---|
| Polyglyceryl-10 stearate / palmitate / behenate | 6.00 | A |
| Cetearyl Alcohol | 0.50 | |
| Glyceryl Stearate | 0.50 | |
| Triethyl Citrate | 2.00 | |
| Ceramide NP | 1.00 | |
| Ceramide AP | 0.50 | |
| Phytosphingosine | 0.50 | |
| Behenic Acid | 0.50 | |
| Cholesterol | 0.50 | |
| Water | 20-25 | B |
| Glycerin | 3.00 | C |
| Water | Ad 100 | |
| Inactivated fermentation broth of Bacillus sp. CECT 5940 | 10-20 | |
| Preservative | 2.00 | D |
| Citric Acid (10 % in water) | pH 5-5.5 | E |

The preparation of the lamellar preparation occurred as follows: All phases were heated to about 90°C. Then phase B was added to phase A with slight stirring, subsequently the mixture A/B was added to phase C with stirring. After homogenizing the mixture A/B/C for 2 minutes with an Ultra Turrax, this mixture was cooled down to 40°C with slight stirring and added to phase D. The mixture A/B/C/D thus obtained is further cooled down to 30°C with slight stirring, while phase E is added.

### Example 5: Testing of the inhibitory activity of the lamellar composition on C. acnes

For testing the inhibitory activity of the lamellar compositions, the inhibition tests were carried out in a similar way like described before in example 3 for the inactivated fermentation broths. Instead of C. acnes subsp. acnes ATCC 6919 the acne-associated strain C. acnes Type 1A-I DSM 1897 was used, which is an isolate from acne lesions of the human facial skin. To test the efficacy of the inhibition, 1:2 and 1:10 dilutions of the liposomal compositions with water were tested in parallel to the inactivated fermentation broth itself and to the undiluted liposomal composition. The results are depicted in the following tables:

**Table 3: Effect of compositions containing heat-inactivated (80°C) fermentation broth (FB) of Bacillus CECT 5940 and ceramides on C. acnes DSM 1897**

| Sample | FB | Comp. without FB | Comp. with 10 % FB | Comp. with 10 % FB diluted 1:2 | Comp. with 10 % FB diluted 1:10 | Comp. with 20 % FB | Comp. with 20 % FB diluted 1:2 | Comp. with 20 % FB diluted 1:10 |
|---|---|---|---|---|---|---|---|---|
| Halo size [mm] | 22 | 0 | 24 | 24 | 21 | 26 | 26 | 23 |

**Table 4: Effect of lamellar compositions containing pH-inactivated (pH3) fermentation broth (FB) of Bacillus CECT 5940 and ceramides on C. acnes DSM 1897**

| Sample | FB | Comp. without FB | Comp. with 10 % FB | Comp. with 10 % FB diluted 1:2 | Comp. with 10 % FB diluted 1:10 | Comp. with 20 % FB | Comp. with 20 % FB diluted 1:2 | Comp. with 20 % FB diluted 1:10 |
|---|---|---|---|---|---|---|---|---|
| Halo size [mm] | 23 | 0 | 24 | 23 | 22 | 25 | 25 | 23 |

As can be seen, the compositions containing a mixture of Bacillus CECT 5940 and ceramide as active ingredients, showed very strong growth-inhibiting activity on the acne-associated C. acnes strain Type 1a-I DSM 1897. The growth-inhibiting activity of the composition was comparable or even a little bit stronger than the growth-inhibiting activity of the fermentation broth itself, despite of the fact that the fermentation broth is used in the compositions only in an amount of 10 or 20 wt.-%. Further, also the diluted lamellar compositions still show very high growth-inhibiting activity against C. acnes DSM 1897, so that the existence of a synergistic effect between the fermentation broth and the ceramides can be assumed. The question, whether the fermentation broth is inactivated by heat or pH treatment is obviously irrelevant, as the results are comparable.

### Example 6: Testing of further B. velezensis strains for activity against C. acnes and S. epidermidis

The positive results as obtained for the strain Bacillus strain CECT 5940, which is currently classified as a *B. velezensis* strain, encouraged the testing of further *B. velezensis* strains for their activity against *C. acnes* and thus the usability of their fermentation broths as ingredient for skin care products. In total 13 *B. velezensis* strains were tested. It was found out that all 13 tested strains without exception showed very strong activity against the acne-associated *C. acnes* strain Type 1A-I DSM 1897 (halo sizes of 12 to 20 mm). Accordingly, strains of the species *B. velezensis* are obviously very suitable as ingredients for skin care products.

### Example 7: Further lamellar formulations containing Bacillus ferment

| Phase | Example | No. 1 | No. 2 | No. 3 | No. 4 | No. 5 | No. 6 | No. 7 |
|---|---|---|---|---|---|---|---|---|
| | | w/w% | w/w% | w/w% | w/w% | w/w% | w/w% | w/w% |
| A | Polyglyceryl-10 stearate / palmitate / behenate | 6.00 | 6.00 | 6.00 | 6.00 | 6.00 | 6.00 | 6.00 |
| A | Ceramide EOP | 0.10 | 1.45 | 0.10 | 0.16 | 0.10 | 0.99 | 0.11 |
| A | Ceramide EOS | | | | | | 0.33 | |
| A | Ceramide NP | 0.51 | | 0.51 | 0.52 | 0.54 | | 0.66 |
| A | Ceramide AP | 0.41 | | 0.41 | 0.10 | 0.45 | | |
| A | Ceramide NS | 0.18 | | 0.18 | 0.29 | 0.22 | | 0.21 |
| A | Cholesterol | 0.47 | 0.42 | 0.47 | 0.47 | 0.45 | 0.32 | 0.49 |
| A | Potassium Cholesterol Sulfate | 0.11 | | 0.11 | 0.11 | | 0.08 | |
| A | N-Acetylphytosphingosine | | | | 0.10 | | | |
| A | Phytosphingosine | | | | | | | 0.22 |
| A | Pentadecanoic acid | | | 0.03 | | | | |
| A | Heptadecanoic acid | | | 0.03 | | | | |
| A | Palmitic acid | 0.11 | 0.08 | 0.09 | 0.11 | 0.14 | 0.08 | 0.15 |
| A | Stearic acid | 0.08 | 0.06 | 0.07 | 0.08 | 0.10 | 0.06 | 0.11 |
| A | Oleic acid | 0.08 | 0.06 | 0.07 | 0.08 | 0.10 | 0.06 | 0.11 |
| A | Linoleic acid | 0.04 | 0.03 | 0.03 | 0.04 | | 0.03 | |
| A | Behenic acid | | | | | 0.12 | 0.08 | |
| A | Carnauba wax acid | 0.12 | 0.09 | 0.10 | 0.12 | | | 0.14 |
| B | Preservative | q.s. | q.s. | q.s. | q.s. | q.s. | q.s. | q.s. |
| C | Water | to 100.0 | to 100.0 | to 100.0 | to 100.0 | to 100.0 | to 100.0 | to 100.0 |
| D | Inactivated fermentation broth of Bacillus sp. CECT 5940 | 10 | 15 | 10 | 20 | 10 | 20 | 15 |

| Phase | Example | No. 8 | No. 9 | No. 10 | No. 11 |
|---|---|---|---|---|---|
| | | w/w% | w/w% | w/w% | w/w% |
| A | Polyglyceryl-10 stearate / palmitate / behenate | 6.0 | 6.0 | 6.0 | 6.0 |
| A | Ceramide EOP | 0.001 | 0.001 | 0.001 | 0.001 |
| A | Ceramide NP | 1 | 1 | 1 | 1 |
| A | Ceramide AP | 0.5 | 0.5 | 0.5 | 0.5 |
| A | Cholesterol | 0.5 | 0.5 | 0.5 | 0.5 |
| A | Phytosphingosine | 0.5 | 0.5 | 0.5 | 0.5 |
| A | Behenic acid | 0.5 | 0.5 | 0.5 | 0.5 |
| A | Salicyloyl Phytosphingosine | | | 0.5 | |
| B | Preservative | q.s. | q.s. | q.s. | |
| C | Creatine | 1.0 | | | |
| C | Panthenol | | 5.0 | | |
| C | Nicotinamide | | | | 5.0 |
| C | Water | to 100.0 | to 100.0 | to 100.0 | to 100.0 |
| D | Inactivated fermentation broth of Bacillus sp. CECT 5940 | 10 | 15 | 10 | 20 |

| Phase | Example | No 12 | No 13 | No 14 |
|---|---|---|---|---|
| | | w/w% | w/w% | w/w% |
| A | Polyglyceryl-10 stearate / palmitate / behenate | 6.00 | 6.00 | 6.00 |
| A | Ceramide EOP | 0.10 | 0.10 | 0.10 |
| A | Ceramide EOS | | | |
| A | Ceramide NP | 0.51 | 0.51 | 0.51 |
| A | Ceramide AP | 0.41 | 0.41 | 0.41 |
| A | Ceramide NS | 0.18 | 0.18 | 0.18 |
| A | Cholesterol | 0.47 | 0.47 | 0.47 |
| A | Potassium Cholesterol Sulfate | 0.11 | 0.11 | 0.11 |
| A | Palmitic acid | 0.11 | 0.11 | 0.11 |
| A | Stearic acid | 0.08 | 0.08 | 0.08 |
| A | Oleic acid | 0.08 | 0.08 | 0.08 |
| A | Linoleic acid | 0.04 | 0.04 | 0.04 |
| A | Behenic acid | | | |
| A | Carnauba wax acid | 0.12 | 0.12 | 0.12 |
| A | Tocopherol | 1.0 | | |
| A | Tocopherol acetate | | 1.0 | |
| A | Retinol palmitate | | | 1.0 |
| B | Preservative | q.s. | q.s. | q.s. |
| C | Water | to 100.0 | to 100.0 | to 100.0 |
| D | Inactivated fermentation broth of Bacillus sp. CECT 5940 | 10 | 20 | 15 |

The formulations are prepared as follows:
Phase A and phase C are heated to 90°C. Phase A is then added to phase C with stirring. This pre-emulsion is then homogenized, for example using an Ultra Turrax. The emulsion is cooled to 40°C with gentle stirring and the preservative (phase B) and the inactivated Bacillus Ferment (phase D) are added. The mixture is then further cooled to 30°C with stirring.

### Example 8: Further formulation examples

| **Ingredient** | **Lamellar Cream (O/W emulsion) Amount [wt.-%]** |
|---|---|
| Dicaprylether | 7.0 |
| Decyl Oleate | 7.0 |
| Behenyl Alcohol | 7.0 |
| Sodium Cetearyl Sulfate | 0.18 |
| Polydimethylsiloxan/Dimethicone | 0.5 |
| Vitamin E-acetate | 1.0 |
| Retinyl palmitate | 1.0 |
| D-Panthenol | 1.0 |
| Ceramide NP | 0.2 |
| Fermentation broth of Bacillus sp. CECT 5940 or B. subtilis DSM 32315 | 1.0 |
| Glycerin | 5.0 |
| Formalin solution (37 %) | 0.08 |
| Water | ad 100 |

| **Ingredient** | **O/W-PIT-Emulsion [wt.-%]** | **W/O-Emulsion [wt.-%]** |
|---|---|---|
| Dicaprylether | 7.5 | 7.0 |
| Decyl Oleate | 7.5 | 7.0 |
| Cetearyl Alcohol | 4.0 | -- |
| Glyceryl palmitate | 2.2 | -- |
| Ceteareth-20 | 2.1 | -- |
| Polydimethylsiloxan/Dimethicone | 0.5 | -- |
| Vitamin E-acetate | 1.0 | -- |
| Retinyl palmitate | 1.0 | 1.0 |
| Biotin | 0.005 | -- |
| Dihydro-3-hydroxy-4,4-dimethyl- 2(3H)-furanone (Pantolactone) | 1.0 | 1.0 |
| Algae extract SPHM 3002 | -- | 1.0 |
| Ceramide AP | 0.2 | 0.2 |
| Fermentation broth of Bacillus sp. CECT 5940 or B. subtilis DSM 32315 | 1.0 | 1.0 |
| Glycerin | 5.0 | 5.0 |
| MgS04 · H20 | -- | 0.7 |
| Formalin solution (37 %) | 0.08 | 0.08 |
| Water | ad 100 | ad 100 |

| **Ingredient** | **Lipoprotein Cream** | **Glycolipid Cream** | **Cream** |
|---|---|---|---|
| Montanov^{®} 202 | -- | -- | 4.0 |
| Thistle oil | 3.0 | -- | -- |
| Evening primrose oil | -- | 3.0 | -- |
| Propylene Glycol Dicaprylate/Dica prate | 3.5 | 3.5 | -- |
| Cocoglycerides | -- | -- | 3.0 |
| Caprylic/Capric Triglyceride | -- | -- | 2.0 |
| Myristyl Myristate | -- | 2.5 | -- |
| Dibutyl Adipate | -- | -- | 7.0 |
| Butyrospermum Parkii (Linne) . | -- | -- | 0.5 |
| Behenyl Alcohol | 3.0 | -- | -- |
| Glyceryl Stearate | 3.0 | 4.0 | 2.0 - |
| Cetearyl Alcohol | 3.0 | 2.0 | 1.0 |
| Isopropyl Stearate | 6.0 | 3.0 | -- |
| Tri-C12-13 Alkyl Citrate | -- | 3.0 | -- |
| Polydimethylsiloxan/ Dimethicone | 1.0 | 1.0 | 0.5 |
| 4-Methylbenzylidene Camphor | 0.6 | 0.6 | 3.0 |
| Butyl Methoxydibenzoylmethane | 0.1 | 0.1 | 2.0 |
| Controx^{®} KS | 0.05 | 0.05 | 0.05 |
| Propylparaben | 0.2 | -- | 0.2 |
| Ceramide NP | 0.2 | 0.1 | 0.2 |
| Inactivated fermentation broth of Bacillus sp. CECT 5940 or B. subtilis DSM 32315 | 1.0 | 1.0 | 1.0 |
| Panthenol | 1.0 | 1.0 | 1.0 |
| Herbasol^{®} distillate malve | -- | 1.0 | -- |
| Herbasol^{®} extract rosmary | -- | -- | 1.0 |
| Aluminium Starch Octenylsuccinate | -- | 3.0 | -- |
| 1,6-Hexanediol | -- | 6.0 | -- |
| Dipropylene glycol | -- | 5.0 | -- |
| Glycerin | 5.0 | -- | -- |
| Dimethylsilanol Hyaluronate | -- | 5.0 | -- |
| V-Protein liquid | 9.0 | -- | -- |
| Titandioxide dispersion | 2.0 | -- | -- |
| Citric acid | 0.1 | -- | -- |
| Sepigel^{®} 305 | 3.0 | 0.4 | -- |
| Hydroxypropyl Methylcellulose | -- | -- | 0.2 |
| Herbasol^{®} distillate green tea | -- | 1.0 | -- |
| Water | ad 100 | ad 100 | ad 100 |

| **Ingredient** | **Mild Cleaning Gel** |
|---|---|
| PEG-40 Hydrogenated Gastor Oil | 0.6 |
| Sodium Cocopolyglucose Tartrate | 2.0 |
| 1,2-Propylene glycol | 10.0 |
| Ceramide EOS | 0.2 |
| Inactivated fermentation broth of Bacillus sp. CECT 5940 or B. subtilis DSM 32315 | 1.0 |
| Bisabolol | 0.1 |
| D-Panthenol | 0-.5 |
| Propylparaben | 0.1 |
| Methylparaben | 0.2 |
| Carbopol^{®} ETD 2020 (0.5%) | 50.0 |
| Water | ad 100 |

| **Ingredients** | Matrix plaster | Matrix plaster |
|---|---|---|
| Polyacrylate Copolymer | 76 | 76 |
| Inactivated fermentation broth of Bacillus CECT 5940 or B. subtilis DSM 32315 | 2.0 | 1.0 |
| Ceramide NP | 0.2 | 0.1 |
| Micrococcus lysate | - | 0.1 |
| Panthenol | 2 | - |
| Dihydro-3-hydroxy-4,4-dimethy\-2(3H)-furanone (Pantolactone) | - | 2 |
| Herbasol^{®} distillate marshmallow | 1 | - |
| Herbasol^{®} distillate green tea | - | 1 |
| Aloe Vera Gel | 1 | 1 |
| Titandioxide dispersion | 2 | - |
| Octocrylene | 1 | - |
| Propylenglycolmonooleat | 5 | 5 |
| Controx^{®} KS | 0.05 | 0.05 |
| Wasser | ad 100 | ad 100 |

| **Ingredients of the gel reservoir** | **Gel Reservoir Plaster** | **Gel Reservoir plaster** |
|---|---|---|
| Inactivated fermentation broth of Bacillus sp. CECT 5940 or B. subtilis DSM 32315 | 1.0 | 1.0 |
| Ceramide AP | 0.1 | 0.15 |
| Ultrasame^{™} | -- | 0.1 |
| Panthenol | 1.0 | -- |
| Pantolacton | -- | 1.0 |
| Bisabolol | 1.0 | 1.0 |
| Herbasol^{®} distillate marshmallow | -- | 1.0 |
| Ethanol | 40 | 40 |
| Polyvinyl alcohol, partially hydrolyzed | 8.0 | 8.0 |
| Polyvinylpyrrolidon | 5.0 | 5.0 |
| Controx^{®} KS | 0.05 | 0.05 |
| Laureth-23 | 2.0 | 2.0 |
| PEG-40 Hydrogenated Gastor Oil | 0.5 | 0.5 |
| Glycerin | 5.0 | 5.0 |
| Water | ad 100 | ad 100 |

### Oil-in-water emulsions

| | **1** | **2** | **3** |
|---|---|---|---|
| Carthamus Tinctorius | 3.00 | 3.00 | 3.00 |
| Caprylic/Capric Triglyceride | 5.00 | 5.00 | 5.00 |
| Cocoglycerides | 2.00 | 2.00 | 2.00 |
| Behenyl Alcohol | 1.00 | 1.00 | 1.00 |
| Glyceryl Stearate | 2.00 | 2.00 | 2.00 |
| Cetearyl Alcohol | 1.00 | 1.00 | 1.00 |
| Isopropylstearate | 4.00 | 4.00 | 4.00 |
| Shea Butter | 2.00 | 2.00 | 2.00 |
| Dimethicone | 1.00 | 1.00 | 1.00 |
| Hydrogenated Palm Glycerides Citrate | 0.05 | 0.05 | 0.05 |
| Propylparaben | 0.20 | 0.20 | 0.20 |
| Cyclopentasiloxane/Dimethiconol | 1.00 | 1.00 | 1.00 |
| Aluminium Starch Octenylsuccinate | 1.00 | 1.00 | 1.00 |
| TiO2 | 0.50 | 0.50 | 0.50 |
| Hexanediol | 6.00 | 3.00 | |
| Propylene glycol | 5.00 | 5.00 | 5.00 |
| Glycerin | 5.00 | 3.00 | 3.00 |
| Methylparaben | 0.20 | 0.20 | 0.20 |
| Sodium Carbomer | 0.40 | 0.40 | 0.40 |
| Dimethylsilanol Hyaluronate | 5.00 | 5.00 | 5.00 |
| Algae Extract | 1.00 | - | - |
| Ceramide NP | 0.1 | 0.2 | 0.1 |
| Ceramide AP | 0.05 | 0.1 | 0.15 |
| Inactivated fermentation broth of Bacillus sp. CECT 5940 or B. subtilis DSM 32315 | 0.5 | 0.8 | 1.2 |
| Perfume | 0.10 | 0.10 | 0.10 |
| Water | ad 100 | ad 100 | ad 100 |
| Cetearyl Isononanoate | 4.00 | 4.00 | 4.00 |
| Mineral oil | 6.00 | 3.00 | 3.00 |
| Glyceryl Stearate / Cetearyl Alcohol / Cetyl Palmitate / Cocoglycerides | 2.00 | 2.00 | 2.00 |
| Palmitic Acid / Stearic Acid | 1.50 | 1.50 | 1.50 |
| Ceteareth-30 | 1.00 | 1.00 | 1.00 |
| Dimethicone | 1.00 | 1.00 | 1.00 |
| Tocoperyl Acetate | 0.50 | 0.50 | 0.50 |
| Propylparaben | 0.30 | 0.30 | 0.30 |
| Sweet Almond Oil | 2.00 | 2.00 | 2.00 |
| Acrylates/C10-30 Alkyl Acrylate Crosspolvmer | 0.27 | 0.27 | 0.27 |
| Glycerin | 5.00 | 3.00 | 3.00 |
| Lactic Acid | 0.26 | 0.26 | 0.26 |
| Propylene glycol | 5.00 | 5.00 | 5.00 |
| Methylparaben | 0.30 | 0.30 | 0.30 |
| Phenoxyethanol | 0.90 | 0.90 | 0.90 |
| Panthenol | 0.50 | -- | -- |
| Laminaria Digitata Extract | 1.00 | -- | -- |
| Sodium Chloride | 0.05 | 0.05 | 0.05 |
| Polyacrylamide / C13-14 Isoparaffin/Laureth-7 | 0.50 | 0.50 | 0.50 |
| Silk Protein | 0.25 | 0.25 | 0.25 |
| Xanthan Gum | 0.20 | 0.20 | 0.20 |
| Ceramide EOP | 0.2 | 0.25 | 0.2 |
| Inactivated fermentation broth of Bacillus sp. CECT 5940 or B. subtilis DSM 32315 | 0.6 | 0.8 | 1.0 |
| Perfume | 0.30 | 0.30 | 0.30 |
| Aqua | ad. 100 | ad. 100 | ad. 100 |
| Hydrogenated Lecithin | 0.50 | 0.50 | 0.50 |
| Isopropylstearate | 4.00 | 4.00 | 4.00 |
| Dibutyl Adipate | 2.00 | 2.00 | 2.00 |
| Tocophervl Acetate | 0.50 | 0.50 | 0.50 |
| Glyceryl Stearate | 1.00 | 1.00 | 1.00 |
| Behenyl Alcohol | 2.00 | 2.00 | 2.00 |
| Dimethicone | 0.50 | 0.50 | 0.50 |
| Propylparaben | 0.20 | 0.20 | 0.20 |
| Cyclomethicone/ Dimethicone Crosspolymer | 1.00 | 1.00 | 1.00 |
| Glycerin | 4.50 | 3.00 | 3.00 |
| Hexandiol | 6.00 | 3.00 | -- |
| Methylparaben | 0.20 | 0.20 | 0.20 |
| Sodium Carbomer | 0.30 | 0.30 | 0.30 |
| Dimethyl Silanol Hyaluronate | 5.00 | 5.00 | 5.00 |
| Algae Extract | 1.00 | -- | -- |
| Plankton Extract | 0.20 | 0.20 | 0.20 |
| Dimethylmethoxy Chromanol | 0.01 | 0.0-1 | 0.01 |
| Propylene glycol | 5.00 | 5.00 | 5.00 |
| Palmitoyl Pentapeptide-3 | 3.00 | -- | -- |
| Palmitoyl Oligopeptide | -- | 2.00 | -- |
| Palmitoyl Tetrapeptide-1 | -- | 1.00 | -- |
| Hydroxyethyl Acrylate/Sodium Acryloyldimethyl Taurate Copolymer / Squalane / Polysorbate 60 | 1.50 | 1.50 | 1.50 |
| TiO2 | 0.50 | 0.50 | -- |
| Ceramide NH | 0.2 | 0.25 | 0.2 |
| Inactivated fermentation broth of Bacillus sp. CECT 5940 or B. subtilis DSM 32315 | 0.5 | 0.9 | 1.3 |
| Perfume | 0.35 | 0.35 | 0.35 |
| Water | ad 100 | ad 100 | ad 100 |

| | **1** | **2** | **3** | **4** | **5** |
|---|---|---|---|---|---|
| Cetearyl Alkohol/ Cetearyl Glucoside | 5.00 | 5.00 | 5.00 | 5.00 | 5.00 |
| Caprylic/Capric Triglyceride | 5.00 | 5.00 | 5.00 | 5.00 | 5.00 |
| Shea Butter | 0.50 | 0.50 | 0.50 | 0.50 | 0.50 |
| Cocoglycerides | 2.00 | 2.00 | 2.00 | 2.00 | 2.00 |
| Cetearyl Alkohol | 1.00 | 1.00 | 1.00 | 1.00 | 1.00 |
| Glyceryl Stearate | 2.00 | 2.00 | 2.00 | 2.00 | 2.00 |
| Dimethicone | 0.50 | 0.50 | 0.50 | 0.50 | 0.50 |
| Tocoperyl acetate | 0.50 | 0.50 | 0.50 | 0.50 | 0.50 |
| Hydrogenated Palm Glycerides Citrate | 0.25 | 0.25 | 0.25 | 0.25 | 0.25 |
| Polysilicone-15 | -- | 4.00 | 4.00 | -- | -- |
| Phenyl benzimidazole Sulfonic Acid | -- | 2.00 | 2.00 | 2.00 | -- |
| Disodium Phenyl Dibenzimidazole Tetrasulfonate | -- | -- | -- | 1.00 | -- |
| Octocrylene | -- | -- | -- | 5.00 | -- |
| 4-Methoxybenzylidene Camphor | 2.00 | -- | -- | -- | -- |
| Butyl Methoxydibenzoylmethane | 1.00 | 1.80 | 1.80 | -- | -- |
| Propylparaben | 0.20 | 0.20 | 0.20 | 0.20 | 0.20 |
| Sodium Carbomer | 0.50 | 0.50 | 0.50 | 0.50 | 0.50 |
| Hexandiol | 6.00 | 3.00 | 3.00 | 6.00 | -- |
| Tale | 0.50 | 0.50 | 0.50 | 0.50 | 0.50 |
| Methylparaben | 0.20 | 0.20 | 0.20 | 0.20 | 0.20 |
| Glycerin | 4.50 | 4.50 | 3.00 | 4.50 | 3.00 |
| Aluminium Starch Octenylsuccinate | 1.00 | 1.00 | 1.00 | 1.00 | 1.00 |
| Hydrogenated Lecithin | 1.(:)0 | ·1.00 | 1-.00 | 1;00 | 1.00 |
| Fagus Silvatica Extract | 2.00 | 2.00 | 2.00 | 2.00 | 2.00 |
| Hydrolyzed Soy Protein | 2.00 | 2.00 | 2.00 | 2.00 | 2.00 |
| Dirnethylsilanol Hyaluronate | 2.00 | 2.00 | 2.00 | 2.00 | 2.00 |
| Trisodium NTA | 0.10 | 0.10 | 0.10 | 0.10 | 0.10 |
| Phenoxyethanol | 0.40 | 0.40 | 0.40 | 0.40 | 0.40 |
| Hydrolyzed Wheat Protein | 3.00 | 3.00 | 3.00 | 3.00 | 3.00 |
| Ceramide NP | 0.25 | 0.2 | 0.15 | 0.2 | 0.25 |
| Inactivated fermentation broth of Bacillus sp. CECT 5940 or B. subtilis DSM 32315 | 0.8 | 1.2 | 1.0 | 1.0 | 1.2 |
| Perfume | 0.40 | 0.40 | 0.40 | 0.40 | 0.40 |
| Water | ad. 100 | ad. 100 | ad. 100 | ad. 100 | ad.100 |
| | | | | | |

| | 1 | | **2** | 3 | |
|---|---|---|---|---|---|
| C14-22 Alcohols/C12-20 Alkyl Glucoside | 3.00 | | 3.00 | 3.00 | |
| Dibutyl Adipate | 6.00 | | 3.00 | 6.00 | |
| Caprylic/Capric Triglyceride | 3.00 | | 3.00 | 3.00 | |
| Cetearyl Alkohol | 1.00 | | 1.00 | 1.00 | |
| Glvceryl Stearate | 0.50 | | 0.50 | 0.50 | |
| Dimethicone | 0.50 | | 0.50 | 0.50 | |
| Propylparaben | 0.20 | | 0.20 | 0.20 | |
| Cyclopentasiloxane | 1.50 | | 1.50 | 1.50 | |
| Glycerine | 5.00 | | 5.00 | 5.00 | |
| Sorbitol | 3.00 | | 3.00 | 3.00 | |
| Methylparaben | 0.20 | | 0.20 | 0.20 | |
| Aluminium Starch Octenylsuccinate | 0.50 | | 0.50 | 0.50 | |
| Sodium Carbomer | 0.10 | | 0.10 | 0.10 | |
| Dimethylsilanol Hyaluronate | 2.00 | | 2.00 | 2.00 | |
| Laminata Digitata Extract | 0.50 | | 0.50 | 0.50 | |
| Hydroxyethyl Acrylate / Sodium Acryloyldimethyl Taurate Copolymer/ Squalene / | 1.00 | | 1.00 | 1.00 | |
| Polysorbate 60 | 1.00 | | 1.00 | 1.00 | |
| Inactivated fermentation broth of Bacillus sp. CECT 5940 or B. subtilis DSM 32315 | 0.4 | | 0.8 | 1.2 | |
| Perfume | 0.20 | | 0.20 | 0.20 | |
| Water | Ad 100 | Ad 100 | Ad 100 | | |

### Water-in-oil Emulsions

| | **1** | **2** | **3** |
|---|---|---|---|
| Polyglyceryl-3 Diisostearate | 3.0 | 3.0 | 3.0 |
| PEG-45/Dodecyl Glycol Copolymer | 0.5 | 0.5 | 0.5 |
| Microcrystalline Wax | 3.0 | 3.0 | 3.0 |
| Bis-Diqlycervl Polyacyladipate-2 | 1.0 | 1.0 | 1.0 |
| Paraffin oil " | 8.0 | 8.0 | 8.0 |
| Vaseline | 2.0 | 2.0 | 2.0 |
| Vitamin-E-acetate | 2.0 | 2.0 | 2.0 |
| Methylparaben | 0.3 | 0.3 | 0.3 |
| Propylparaben | 0.3 | 0.3 | 0.3 |
| Isopropyl isostearate | 8.0 | 8.0 | 8.0 |
| Glycerin | 5.0 | 3.0 | 3.0 |
| Mg-Sulfate | 0.5 | 0.5 | 0.5 |
| Ceramide EODS | 0.2 | 0.1 | 0.15 |
| Inactivated fermentation broth of Bacillus sp. CECT 5940 or B. subtilis DSM 32315 | 0.8 | 1.2 | 1.5 |
| Lactic acid 80% | 0.560 | 0.560 | 0.560 |
| Algae Extract | 1.0 | -- | -- |
| Panthenol | 0.5 | 1.0 | 0.5 |
| Calendula Officinalis Flower Extract | 0.3 | -- | -- |
| Propylene Glycol | 0.5 | -- | -- |
| Parfum | 0.2 | 0.2 | 0.2 |
| Water | ad 100 | ad 100 | ad 100 |

### Cleaning preparations

| | **1** | **2** | **3** |
|---|---|---|---|
| Dipropylene qlycol | 10.00 | 10.00 | 10.00 |
| Chlorhexidine digluconate | 1.00 | 1.00 | 1.00 |
| Poloxamer-184 | 3.00 | 3.00 | 3.00 |
| Panthenol | 0.50 | 0.50 | 0.50 |
| Chitosan Glycolate | 3.00 | 3.00 | 3.00 |
| PEG-40 Hydrogenated Castor Oil / Trideceth 9 / Propylene Glycol | 0.50 | 0.50 | 0.50 |
| Chlorella Vulgaris Extract | 0.50 | 0.50 | 0.50 |
| Ceramide NP | 0.15 | 0.1 | 0.2 |
| Inactivated fermentation broth of Bacillus sp. CECT 5940 or B. subtilis DSM 32315 | 0.4 | 0.8 | 1.2 |
| Perfume | 0.20 | 0.20 | 0.20 |
| Water | ad. 100 | ad. 100 | ad. 100 |
| | | | |

| | **1** | **2** | **3** |
|---|---|---|---|
| Carbomer | 1.40 | 1.40 | 1.40 |
| Sorbitol | 2.10 | 2.10 | 2.10 |
| Sodium Benzoate | 0.40 | 0.40 | 0.40 |
| Laurvl Glucoside | 7.50 | 7.50 | 7.50 |
| Cocoamidopropyl betaine | 3.40 | 3.40 | 3.40 |
| Disodium Laureth Sulfosuccinate | 5.00 | 5.00 | 5.00 |
| PEG-7 Glyceryl Cocoate | 0;50 | 0.50 ⁻ | 0.50 ⁻ -- |
| Coco-Glucoside / Glyceryl Oleate | 5.00 | 5.00 | 5.00 |
| Hydrogenated Palm Glycerides Citrate | 0.05 | 0.05 | 0.05 |
| Sodium PCA | 1.60 | 1.60 | |
| Pantolactone | 1.00 | 1.00 | |
| Tetrasodium EDTA | 0.25 | 0.25 | 0.25 |
| Sodium Lactate | 1.80 | | |
| Panthenol | 0.50 | 0.50 | 0.50 |
| Ceramide NP | 0.1 | 0.15 | 0.25 |
| Inactivated fermentation broth of Bacillus sp. CECT 5940 or B. subtilis DSM 32315 | 0.8 | 1.2 | 1.6 |
| Perfume | 0.40 | 0.40 | 0.40 |
| Aqua | ad. 100 | ad. 100 | ad. 100 |

### Water-in-Silicone Emulsions

| | **1** | **2** | **3** |
|---|---|---|---|
| Dimethicone | 2.50 | 2.50 | 2.50 |
| Cyclomethicone | 25.00 | 25.00 | 25.00 |
| Cetyl Dimethicone Copolyol | 2.00 | 2.00 | 2.00 |
| Sodium Chloride | 2.00 | 2.00 | 2.00 |
| Perfume | 0.30 | 0.30 | 0.30 |
| Chlorhexidine digluconate | 0.20 | 0.20 | 0.20 |
| Ceramide AP | 0.10 | 0.15 | 0.15 |
| Inactivated fermentation broth of Bacillus sp. CECT 5940 or B. subtilis DSM 32315 | 0.4 | 0.8 | 1.2 |
| Water | ad 100 | ad 100 | ad 100 |

### Table of named ingredients:

| **Name** | **INCi** |
|---|---|
| Algenextrakt SPHM 3002 | Aqua, Algae (Linne) |
| Carbopol^{®} ETD 2020 (0.5%ig) | Acrylates/C10-30 Alkyl Acrylate Cross polymer |
| Controx^{®} KS | Tocopherol, Hydrogenated Palm Glycerides Citrate |
| Montanov^{®} 202 | Arachidyl Alcohol, Behenyl Alcohol, Arachidyl Glucoside |
| Sepigel^{®} 305 | Polyacrylamide, C13-14 Isoparaffin, Laureth-7 |
| V-Protein liquid cos 152/22 (Cosmetochem) | Aqua, Propylene Glycol, Hydrolyzed Pea Protein (Pisum Sativum) |

## Claims

1. Cosmetical composition containing at least one Bacillus strain or a fermentation broth thereof and at least one ceramide.

2. Cosmetical composition according to claim 1, wherein the composition is a topical skin care or a dermatological composition.

3. Pharmaceutical composition for topical or dermatological applications, in particular for treating or preventing a disease associated with pathogenic Cutibacterium acnes and/or for treating or preventing acnes vulgaris, dermatitis, Rosaceae or Couperose, containing at least one Bacillus strain or a fermentation broth thereof and at least one ceramide.

4. Composition according to any of the preceding claims, wherein the ceramide is selected from ceramide NP, ceramide AP, ceramide EOP, ceramide NDS, ceramide ADS, ceramide EODS, ceramide NS, ceramide AS, ceramide EOS, ceramide NH, ceramide AH, ceramide EOH and mixtures thereof, preferably from ceramide NP, ceramide AP, ceramide NS, ceramide EOP, ceramide EOS and mixtures thereof, above all from ceramide NP, ceramide AP and mixtures thereof.

5. Composition according to any of the preceding claims, wherein the Bacillus strain is selected from the species *B. subtilis, B. amyloliquefaciens, B. velezensis, B. licheniformis, B. paralicheniformis* and *B. pumilus,* more preferably from the species *Bacillus subtilis, B. amyloliqufaciens* and *Bacillus velezensis,* above all from the species *B. subtilis* and *B. velezensis.*

6. Composition according to any of the preceding claims, wherein the Bacillus strain is selected from *B. velezensis*/*B. amyloliquefaciens* CECT 5940 and *B. subtilis* DSM 32315.

7. Cosmetical composition containing the Bacillus strain CECT 5940 or the *B. subtilis* strain DSM 32315 or a mutant of such strains or a fermentation broth thereof, wherein the mutants have a sequence identity of at least 99 %, preferably at least 99.5 %, more preferably at least 99.9 % with respect to the genomic DNA of the parent strains.

8. Composition according to any of the preceding claims, wherein the composition is an emulsion, in particular a W/O or an O/W emulsion, preferably a micellar, a lamellar or a liposomal composition, more preferably a lamellar composition.

9. Composition according to any of the preceding claims, wherein the Bacillus strain or fermentation broth thereof has growth-inhibiting activity against at least one skin pathogen, preferably against *C. acnes,* and has preferably no growth-inhibiting activity against *S. epidermidis.*

10. Composition according to any of the preceding claims, wherein more than 80 %, preferably more than 90 %, more preferably all of the Bacillus strains as contained in the composition are non-viable and/or preferably less than 10 %, more preferably less than 5 %, above all none of the Bacillus cells are present as spores.

11. Composition according to any of the preceding claims, wherein the composition contains at least one non-ionic emulsifier, preferably at least one polyglycerol carboxylic acid ester, more preferably at least two polyglycerol carboxylic acid esters, wherein the polyglycerol group of the polyglycerol carboxylic ester has preferably a degree of polymerization of 2.0 to 25, more preferably of 2.5 to 20, in particular of 3.0 to 15, and the carboxylic acid group is preferably a fatty acid, in particular having 12 to 26, preferably 14 to 24, more preferably 16 to 22 carbon atoms.

12. Composition according to any of the preceding claims, wherein the composition contains at least one further ingredient selected from phospholipids, sphingoid bases, free fatty acids, surfactants, protein hydrolyzates, polymers, light stabilizers, anti-perspirants, deodorants, peptides, amino acids, vitamins, extracts from plants and algae, cosmetic oils, emollients, antioxidants, preservatives, thickeners, viscosity regulators, stabiliziers, hydrotropes, solids, fillers, film formers, conditioners, insect repellents, self-tanning agents, odour absorbers, solvents, perfumes and dyes.

13. Method of preparing a composition according to any of the claims 1 to 12, comprising the following steps:
a) Providing a fermentation broth of Bacillus cells with growth-inhibiting activity against skin pathogens, in particular *C. acnes,* wherein the Bacillus cells preferably have no growth-inhibiting activity against *S. epidermidis,*
b) Killing all or the major part of the Bacillus cells, preferably by heat treatment and/or pH shift,
c) Mixing the fermentation broth thus obtained with substances suitable for cosmetical skin applications.

14. Use of a Bacillus strain or of a fermentation broth thereof or of a cosmetical composition according to any of the preceding claims, wherein the Bacillus strain is preferably the Bacillus strain CECT 5940, the Bacillus strain DSM 32315 or a mutant of such strains, for treating the human skin topically, in particular for treating irritated, impure, dry, blemished or acne-prone human skin and/or for beneficially modulating the skin microbiome or the immune status of the skin and/or for avoiding or reducing erythema or inflammation of the skin and/or for increasing the barrier function of skin.

15. Non-therapeutical method of treating the human skin, in particular of treating irritated, impure, dry, blemished or acne-prone human skin and/or of beneficially modulating the skin microbiome or the immune status of the skin and/or of avoiding or reducing erythema or inflammation of the skin and/or for increasing the barrier function of skin, wherein a Bacillus strain or a fermentation broth thereof or a cosmetical composition according to any of the preceding claims is applied to the human skin, wherein the Bacillus strain is preferably the Bacillus strain CECT 5940, the Bacillus strain DSM 32315 or a mutant of such strains.

16. Bacillus strain or fermentation broth thereof for use in a method of treating or preventing a C. *acnes* associated disease of the human skin and/or in a method of treating acne vulgaris, dermatitis, Rosaceae or Couperose, wherein the Bacillus strain is preferably the Bacillus strain CECT 5940, the Bacillus strain DSM 32315 or a mutant of such strains.
